# EUROPEAN PATENT SPECIFICATION

(11) **EP 0 848 617 B1**
(45) Date of publication and mention of the grant of the patent: **16.05.2001**
(21) Application number: 96928236.7
(22) Date of filing: 17.08.1996
(51) Int. Cl.: A61L 2/10, A61L 9/20

(54) **METHOD AND APPARATUS FOR KILLING MICROORGANISMS**
VERFAHREN UND VORRICHTUNG ZUR ABTÖTUNG VON MIKROORGANISMEN
PROCEDE ET DISPOSITIF POUR TUER LES MICRO-ORGANISMES

(30) Priority: 30.08.1995 US 521527
(43) Date of publication of application: 24.06.1998
(73) Proprietor: Katz, Janyce, C., Columbus, OH 43209 (US); Glazman, Mark, Columbus, OH 43209 (US)
(72) Inventor: GLAZMAN, Mark, Columbus, OH 43209 (US)
(74) Representative: Weyland, J.J. Pierre
(86) International application number: PCT/US96/13417
(87) International publication number: WO 97/07831

(56) References cited:
- EP-A- 0 011 776
- EP-A- 0 351 518
- EP-A- 0 461 310
- WO-A-87/06841
- WO-A-90/04454
- WO-A-94/08633
- US-A- 5 112 370

## Description

### FIELD OF THE INVENTION

The present invention relates to fluid purification and in particular sterilization by irradiation with an ultraviolet radiation source.

### BACKGROUND OF THE INVENTION

The airborne transmission of bacteria and viruses, chiefly respiratory disease organisms is a serious problem in health care. The control of airborne disease transmission has become increasingly important with an increasing number of people growing older with weakened immune systems more vulnerable to airborne disease or infected with human immunodeficiency virus (HIV) or other airborne and difficult to cure diseases. This coupled with antibiotic resistant strains of bacteria have created a need for inexpensive, efficient air purification systems. The spread of air born infections can be reduced by killing the infectious microorganism by ultraviolet (UV) radiation. Ultraviolet radiation to destroy airborne microorganisms can be used in ceiling fixtures suspended above the people in the room or inside ventilation system air duct.

The continuing spread of tuberculosis (TB) infection and other airborne disease in modern health institutions, correctional institutions, and shelters for homeless indicates however, that the known air purification systems are inadequate in controlling the spread of airborne microorganisms.

An other important field where the spread of microorganisms needs to be controlled is liquid, and particularly waterbased solutions.

The sterilization by ultraviolet radiation has been known more than fifty years. Various methods and apparatus have been invented for ultraviolet irradiating fluids, air and water in particular, in order to control the spread of microorganisms by destroying those microorganisms with a sufficient dose of radiation.

Air purification by means of filtration and irradiation is widely practiced. Conventional air cleaning systems commonly have a filtration and irradiation units. Irradiation is placed after filtration because the ultraviolet lamps used as a source of the radiation readily attract dust which can accumulate on a surface of the lamp, block the UV radiation inside the lamp and interfere with their germicidal effect .

Commonly irradiation is placed before humidification because ultraviolet radiation is most effective in an atmosphere with relative humidity less than 70% which promotes oxidation. Ultraviolet germicidal radiation has been proven to be more effective and economically feasible than any other approach to reducing the number of microorganisms in the liquid or gas flow.

Conventional UV fluid sterilization systems have relied on exposure of suspended microorganisms to ultraviolet radiation by passing medium over or around one or more ultraviolet lamps. This method is used in US patents 5,112,370 and 5,200,156 which corresponds to PCT application WO-A-900445. This method has a number of shortcomings.

The first shortcoming of the previous art is their low reliability. The particles suspended in the fluid accumulate on the surface of the lamp or protective tubes, forming the UV light absorption layer, which restricts or eliminates the germicidal effectiveness. The reliability and actual germicidal effectiveness depend on the quality of the medium filtration and come very small and unpredicted if the medium is unfiltered or poorly filtered.

The second shortcoming of previous art of UV sterilization systems is that they have low efficiency of use of the UV energy, because their lamps accumulate particles on the surface from the beginning and because in ducts or pipes with ratio length-L to diameter-D L/D=10:1 only 6% of beams have their path length equal to the longest available way(L/2 that is when the lamp is placed halfway between the longest straight line length of the duct (L), the maximum available way is only L/2), other beams, 94% are directed on much shorter paths and could irradiate smaller volume on its way and hence less efficient.

The third shortcoming of previous art is nonuniform irradiation intensity in an irradiated volume. In the device for sterilization according to US patent 5,200,156 the author tried to achieve more uniform irradiation intensity than before by applying a flat oval cross section light source with or without the reflectors. But this invention made limited progress because the according to the US patent 5,200,156 can irradiate towards axis of pipe only 50% of radiation and only 6% of the beams will have length equal to the length of the longest available way. Other beams are short slanting beams. They irradiate smaller volume than longest beams and are absorbed by the pipe walls. Due to the early absorption, the efficiency of the use of short slanting beams is very low. As a result the efficiency of all previous art, including the sterilizer according to US patent 5,200,156 is too low.

The fourth shortcoming of previous art according to US patent 5,200,156 is that the sources of radiation are installed inside the medium flow, liquid or gas, and create a substantial pressure loss in the system. To retrofit an operating ventilation or other system with known UV sterilization system it is necessary to replace a fan, pump, electric motor by more powerful ones. As a result capital and operating expenses would increase.

The prior art therefore suffers from number of disadvantages which can be improved upon.

### SUMMARY OF THE INVENTION

It is an object of the present invention to provide a method for killing microorganism in a flowing fluid medium using germicidal beams as a means for killing the microorganisms in a straight portion of the flow path, the method has the steps as defined in Claim 1.

The advantage of the present invention is the provision of the secondary flow that runs along the surfaces immersed in the fluid means for transferring and prevents accumulation on the surfaces the particles suspended in the fluid medium. This advantage makes the reliability of the method and apparatus according to the present invention high and predictable.

It is a further object of invention to provide filtering of the secondary flow.

It is a further object of invention to energize the means for killing microorganisms, by a means for energizing, having one or more arcs of a ultraviolet lamps for emitting ultraviolet germicidal beams.

It is a further object of invention to orient the emission of the ultraviolet beams into a substantially parallel array of beams and to pass the fluid medium along a straight portion of the flow path aligned with the array of parallel ultraviolet beams, the straight portion of the flow path being of sufficient length to allow the array of ultraviolet beams to kill microorganisms.

An other advantages of the present invention is the maximum uniformity of the irradiation intensity in an irradiated volume, because the beams were oriented in a substantially parallel array, which is parallel the fluid flow and will not suffer from early absorption by walls. The efficiency of use of ultraviolet energy of the ultraviolet lamps will reach the maximum because according to the present invention the maximum amount of UV beams was oriented parallel and because they have the length equal to the longest available way.

According to the preferred embodiment of the invention a substantially parabolic reflector is provided around each the ultraviolet lamp. The arc of the lamp is situated in the focus of the reflector. As a result the maximum amount of the ultraviolet lamp emission is oriented in the parallel array of beams. According to the other preferred embodiment, each of the reflectors is provided with an aperture for accepting at least a portion of the secondary flow of substantially particulate free fluid; and passing the portion of the secondary flow of fluid through each reflector. As a result, the surfaces of the reflector and the lamp will remain clean and provide maximum transfer of radiation to the fluid medium.

According to an other preferred embodiment a fluid impenetrable wall is provided as a means for transferring which can allow the irradiation for the means for killing microorganisms to pass or be transmitted. The wall imperviously separates the fluid medium from the means for orienting and the means for energizing. This embodiment could be applied when the fluid medium is liquid or gas. The additional advantage of this embodiment is a safe and convenient maintenance of the lamps and reflectors because they are located outside of the fluid medium.

It is a further object of invention to provide an apparatus for killing microorganisms as defined in Claim 10.

Preferably the conduit means for passing the secondary flow of the fluid medium has a filter, the filter being sufficient to remove particles from the secondary flow. The secondary flow is a small part of the fluid medium and the filter to remove particles the secondary flow is also small and inexpensive. The flow through the filter is very small and has the velocity 0.1-0.25 m/sec, (20-50 FPM) and its life time is a few times longer than the life time of a filter which conventionally is used for the filtration of the prime flow with the velocity 1.27 m/sec(250 FPM). As a result the means for transfer remain clean independently of the purity of the fluid medium, the period between the maintenances is much longer, and the reliability of the apparatus is higher than previously known arts.

Preferably the means for energizing has one or more arcs of ultraviolet lamps for emitting ultraviolet beams. The lamps emit an ultraviolet radiation in the medium and are well known as the most effective source of germicidal radiation.

Preferably the apparatus comprises at least one means for orienting the emission of the ultraviolet beams into a substantially parallel array of beams. The substantially parallel array of the beams provides most effective use of the ultraviolet energy.

Preferably the means for orienting the ultraviolet beams is a substantially parabolic reflector with the arc of ultraviolet lamp situated in its focus.

Preferably a conduit means for passing the fluid medium along a path aligned with the array of parallel ultraviolet beams is provided, the path being of sufficient length to allow the array of beams of ultraviolet radiation to kill microorganisms.

Preferably the conduit means for passing the fluid medium is a straight prime conduit. The means of orienting is situated at the end of the straight prime conduit and is faced at the straight prime conduit.

According to preferred embodiment the substantially parabolic reflector having an aperture open to the secondary flow is used as the means for orienting. The substantially parabolic reflector preferably is open to the straight prime conduit at a first end, at a second end, the substantially parabolic reflector is open for passing of the secondary flow of the fluid medium. The secondary flow is running along the substantially parabolic reflector and the lamp located in the reflector cavity and establishing a substantially particulate free barrier environment maintaining the surfaces of the substantially parabolic reflector and lamp clean.

The advantages of the invention can be seen in Table 1 as a result of a comparison of the units for air sterilization in the ducts 0.89m x 1.02m x 2.54 m (35"x 40"x100") with airflow 50.67 m3/sec ( 10000 CFM), temperature 26.6 °C (80°F). The source of germicidal radiation is the germicidal lamps G36T6H with ultraviolet output 13.8 W per lamp. Unit 1 is a unit according to the previous art with two rows of the lamps installed across to the air flow. Unit 2 is the unit according to this invention.

**Table 1**

| Number of the unit | Number of the lamps in the unit | Size of the unit, m | Wattage of the unit UV, Watt | Relative air flow resistance | Relative efficiency at the beginning of the operation | Relative efficiency after one month of the operation |
|---|---|---|---|---|---|---|
| 1 | 47 | 0.89x1.02x0.91 | 662 | 47 | 100 | 20 |
| 2 | 16 | 0.89x1.02x0.41 | 226 | 1 | 100 | 100 |

The relative air-flow resistance is the ratio between the coefficient of pressure losses in the unit 1 and the coefficient of pressure losses in the unit 2. Relative efficiency is the ratio between the efficiency of the unit at present time and the efficiency of the unit at the initial moment of operation. The efficiency is defined from the equation N/No where No is the number of microorganisms in the medium before the treatment and N is the number of inactivated microorganisms in the media after the treatment. The units are the percentage.

The unit 2 is estimated to be about five times more efficient after one month of operation, uses three times less ultraviolet lamps and energy, and has very low air flow resistance.

According to another preferred embodiment, the means for transferring is fluid impenetrable, germicidal radiative transmissible wall, imperviously covering the end of the straight prime conduit, wherein the means for killing of microorganisms enter in and separated the fluid flow from means for orienting and means for energizing. The fluid impenetrable wall separates the compartment, wherein the substantially parabolic reflector and lamp are located. This embodiment could be preferable when the fluid is liquid or when fluid flow has to be separated from the means of energizing and the means of orienting. The advantages of the present invention is the provision of the location of means for energizing and means for transferring outside of the fluid flow that is providing the convenience and safe maintenance of a electrical contacts of the lamps and surfaces of the lamps and reflectors.

### BRIEF DESCRIPTION OF THE DRAWINGS

The invention will now be explained by way of example only and with reference to the following drawings wherein:
FIG. 1 is a schematic view of a first preferred embodiment of the invention showing an apparatus for killing microorganisms in a fluid medium with a vertical cross-sectional view taken along axis of the means for passing the fluid medium to expose the components of the apparatus for killing microorganisms;
FIG. 2 is a vertical cross-sectional view taken along lines 2-2 of the apparatus for killing microorganisms shown in FIG. 1, illustrating the means for transferring and means for energizing components of the apparatus for killing microorganisms;
FIG. 3 is a schematic view of a second preferred embodiment of the invention showing the apparatus for killing microorganisms with two lamp light source;
FIG. 4 is a schematic view of the third preferred embodiment showing the apparatus for killing microorganisms with a separated radiative compartment.
FIG. 5 is a cross-sectional view of an fourth embodiment made in accordance to the invention.

### DETAILED DESCRIPTION OF THE PREFERRED EMBODIMENT

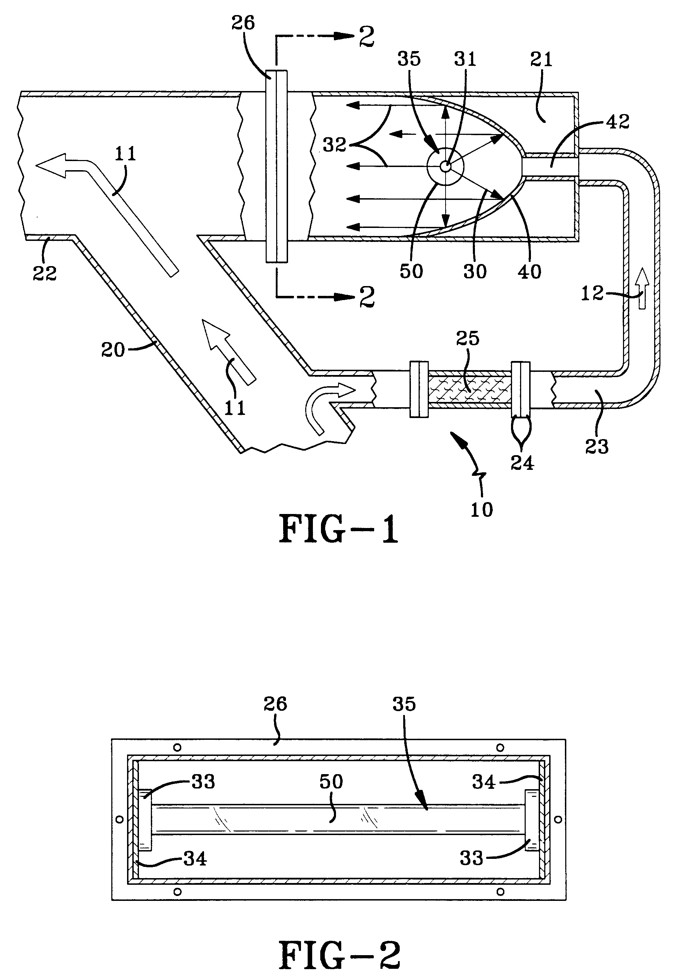

The present invention provides the apparatus for killing microorganisms, generally referred to by reference 10, 100. First preferred embodiment of the invention is shown in FIG. 1 and FIG. 2. The means for passing the fluid medium is conduit 20, having the straight prime conduit 22. The beginning portion of the straight prime conduit 22 is connected with dead end chamber 21, by flanges 26. The dead end chamber 21 is open towards the flow of the fluid medium 11, and faces towards the straight prime conduit 22.

The means for transferring is the envelope 50 of the ultraviolet lamp 35. The lamp 35 includes the glass envelope 50, the arc 31, electrodes and electrical contacts as is understood by those of ordinary skill of the art. The parabolic reflector 40 is the means for orienting. The means for energizing is an arc 31 of the ultraviolet lamp 35 enclosed in a transparent for ultraviolet radiation envelope 50. The parabolic reflector 40 is located inside dead end chamber 21. The arc 31 of the ultraviolet lamp 35 is situated in the focus of the parabolic reflector 40 by the lampholders 33. The lampholders 33 are mounted on the side panels 34. The parabolic reflector 40 is installed such that its axis or axis plane is parallel to the axis or axis plane of the straight prime conduit 22. The parabolic reflector 40 has an aperture 42.

According to the first preferred embodiment, the means for passing the secondary flow include a secondary conduit 23 with a filter 25 installed by flanges 24. The intake end of the secondary conduit 23 is connected and open to the conduit means for passing of the fluid medium 20. The outlet of the secondary conduit is connected with the dead end chamber 21. The filter 25 is an effective particulate filter. In case of the air filtration the high efficiency particulate filters or electrostatic filter could be used but the application of other filters is not limited.

According to the first preferred embodiment, the method and apparatus for killing microorganisms in the fluid media is realized as follows:
The fluid medium 11 is coming through the conduit means for passing the fluid medium, conduit 20 and entering in the straight prime conduit 22. The secondary flow medium 12 is a small part of the fluid medium 11 and goes through the conduit means for passing the secondary flow, conduit 23 including the effective particulate filter 25. The filter 25 captures and arrests the particles suspended in the secondary flow medium 12. The clean secondary flow medium, coming in the end of the chamber 21 and through the aperture 42, contacts the reflector 40, and the lamp envelope 50, fills up the cavity of the reflector 40 and protects the lamp envelope 50 and the reflector 40 from the accumulation of the particles from the flow of the fluid medium 11.
At the same time the arc 31 of the ultraviolet germicidal lamp 35 emits the means for killing microorganisms, the means being germicidal beams 30. The means for transferring, the lamp envelope 50 transfers the germicidal beams 30 to the means for orienting the parabolic reflector 40. Due to the parabolic shape and the situation of the arc 31 in the focus of the parabolic reflector 40, the parabolic reflector is the means for orientation. The parabolic reflector orients the germicidal beams 30 into a substantially parallel array of ultraviolet beams 32. The straight prime conduit 22 pass the fluid medium 11 along a path aligned with the array of the substantially parallel ultraviolet beams 32.

The substantially parallel array of the ultraviolet beams maximizes and uniformly radiates the fluid 11 passing the straight prime conduit 22. The microorganisms suspended in the fluid absorb the substantially parallel arrays of beams 32, are killed before passing the end of the straight prime conduit 22.

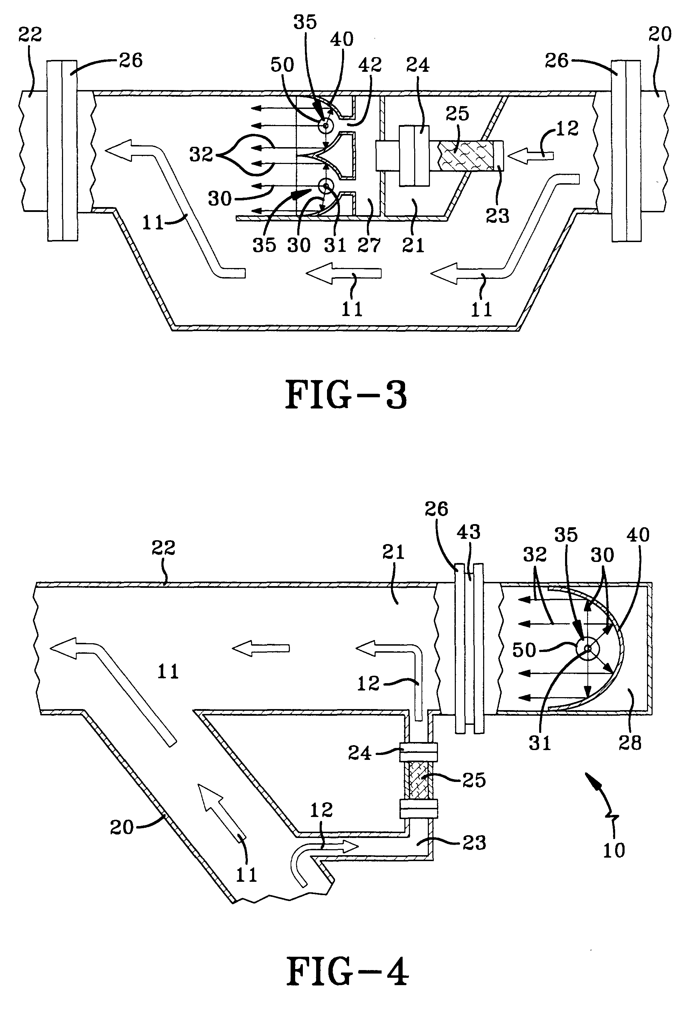

The second preferred embodiment is shown in Fig 3. The apparatus 10 has the dead end chamber 21, which is installed inside the flow of the fluid medium 11. The dead end chamber 21 contains two parabolic reflectors 40, the germicidal lamps 35 with arcs 31 situated in focuses of the reflectors 40, a receiver 27 and the conduit means for passing the secondary flow 23. The number of lamps and reflectors 40 is not limited to two. If it is necessary a larger number of reflectors 40 could be situated in the dead end chamber 21. The fluid medium 11 flows in the apparatus for killing microorganisms 10, runs along outside the dead end chamber 21 and continues to move along the straight prime conduit 22. The secondary flow medium 12 is a small part of the fluid medium 11 and goes through the effective particulate filter 25. The filter 25 captures and arrests particles suspended in the secondary flow medium 12. Clean secondary flow medium comes in the receiver 27. The receiver 27 is a chamber with impenetrable walls having input to the clean secondary flow of the fluid medium and output connected with the apertures 42 of the parabolic reflectors 40. The clean medium comes through the aperture 42 fills up the cavity of the reflector 40 and protects the lamp envelopes 50 and the reflectors 40 from accumulation of the particles from the fluid medium 11.

At the same time the arcs 31 of the ultraviolet germicidal lamps 35 emit the germicidal beams 30. The means for transferring, the lamp envelop 50 transfers the germicidal beams 30 to the means for orienting the parabolic reflectors 40. Each parabolic reflector 40 orients the germicidal beams 30 in the substantially parallel array of the ultraviolet beams 32. The straight prime conduit 22 passes the fluid medium 11 along a path aligned with the array of the substantially parallel ultraviolet beams 32.

The substantially parallel array of the ultraviolet beams 32 maximizes and uniformly radiates the fluid 11 passing through the straight prime conduit 22. The microorganisms suspended in the fluid absorb the substantially parallel arrays of beams, and are killed in the straight prime conduit 22.

The third preferred embodiment is shown on Fig. 4 and includes the conduit means for passing the fluid medium, the conduit 20, having the straight prime conduit 22. The beginning of the straight prime conduit 22 is connected with the dead end chamber 21. The dead end chamber 21 opens towards the flow of the fluid medium 11, and faces towards the straight prime conduit 22. At the closed end of the dead end chamber 21 is a transmissible wall 43, transferring the means for killing and impenetrable for the fluid medium 11. The transmissible wall 43 separates the fluid medium 11 from the parabolic reflectors 40, lamp envelopes 50 and electrical connectors located in the radiative chamber 28.

The means for transferring are the transmissible wall 43 and the envelope 50 of the ultraviolet lamp. The parabolic reflector 40 is the means for orienting. The means for energizing is the arc 31 of the ultraviolet lamp enclosed in a transparent for ultraviolet radiation envelope 50. The parabolic reflector 40 is located inside the radiative chamber 28. The arc 31 of the ultraviolet lamp is situated in the focus of the parabolic reflector 40. The parabolic reflector 40 is installed such that its axis or axis plane is parallel to the axis or axis plane of the straight prime conduit 22.

According to the third preferred embodiment the conduit means for passing the secondary flow include a secondary conduit 23 and a filter 25 installed by flanges 24. The intake end of the secondary conduit 23 connected and open to the conduit means for passing of the fluid medium 20. The outlet of the secondary conduit is connected with the dead end chamber 21. The filter 25 is effective particular filter. The filter 25 captures and arrests particles suspended in the secondary flow medium 12. Clean secondary flow medium coming in the dead end chamber 21 and through the aperture 42 runs along the transmissible wall 43, fills up the dead end chamber 21 and protects the transmissible wall 43 from accumulation of the particles from the flow of the fluid medium 11.

At the same time the arc 31 of the ultraviolet germicidal lamp emits the germicidal beams 30. The means for transferring, the lamp envelopes 50 transfers the germicidal beams 30 to the means for orienting the parabolic reflectors 40. The parabolic reflector orients germicidal beams 30 in the substantially parallel array of the ultraviolet beams 32. The straight prime conduit 22 passes the fluid medium 11 along a path aligned with the array of the substantially parallel ultraviolet beams 32.

The substantially parallel array of the ultraviolet beams 32 pass through the transmissible wall 43, maximizing and uniformly radiating the fluid 11 passing the straight prime conduit 22. The microorganisms suspended in the fluid absorb the substantially parallel arrays of beams are killed prior to passing the end of the straight prime conduit 22. For additional increasing of efficiency in an outlet end of the straight prime conduit 22 a flat reflector could be installed. The flat reflector should also be maintained clean by a secondary flow of substantially particles free fluid 11 as described above; the secondary flow running along or flowing over the surface of the reflector, creating a barrier of a particle free media or flow.

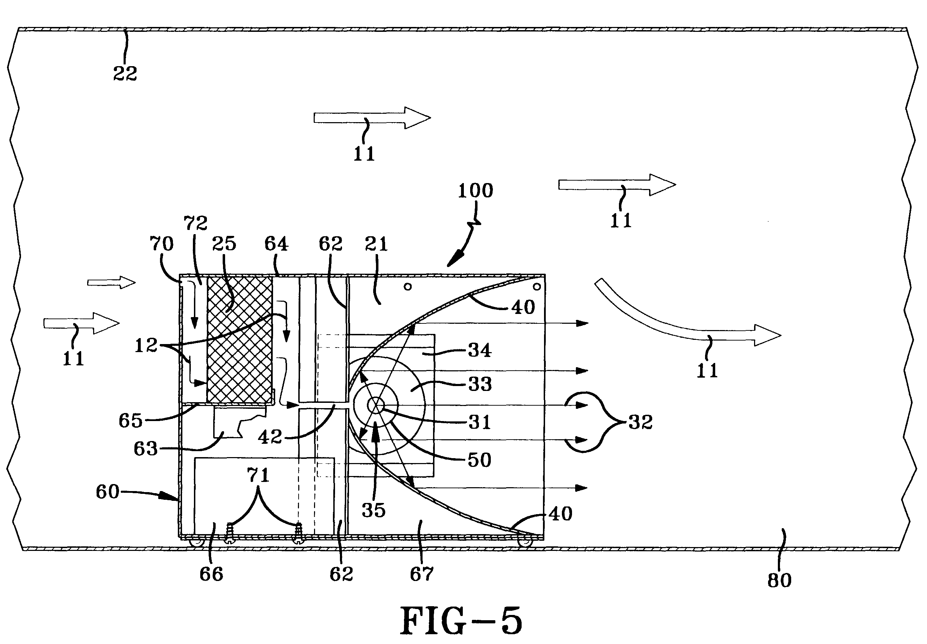

As shown in FIG. 5, a fourth preferred embodiment is disclosed. This apparatus 100 has a compact size and is suitable for placing in the ducting 80 of heating, airconditioning, and ventilation systems.

The apparatus 100 has a housing 60, a pair of walls 62, the walls 62 being spaced apart a small distance thus providing an air path or slit for the secondary flow of air to pass through, a filter holder bracket 63, a filter shelf 65, a removable cover 64, the cover 64 being adapted to hold the filter 25 firmly and air tightly against the shelf 65 of the appratus 100. As shown the housing 60 is in the form of a box, the filter 25 is snugly and airtighly pressed into the housing, the ends of the filter 25 being compressed against walls or sides 67 of the housing 60. In this way the perimeter sides of the filter are tightly sealed against the sides 67, the shelf 65 and the cover 64. A small opening 70 allows the air from a primary flow 11 of fluid medium containing microorganisms and particles to be separated into a secondary flow 12. This secondary flow is ducted to the filter 25 via a small chamber 72 facing the filter 25. The air of this secondary flow 12 passing through this particle filter of a filtration size recommended to be like a "HEPA" type, effective for capturing dust particles of a size 0.3 micron or bigger. This filtered air 12 then passes through the slit or running between the walls 62 and fills the space defined by the reflector 40 by passing through the aperture 42 of the reflector 40 and over the glass envelope 50 of the lamp 35 thus providing a particle-free barrier of secondary flow 12 protecting the surfaces of these transferring means.

As shown the apparatus 100 further includes a ballast 66 which is attached to the housing by the threaded fasteners 71. The ballast 66 assists in energizing the lamp 35. Not shown is the associated wiring and connections needed to provide electricity to the ballast and ultimately the lamp as these features are commonly understood by those of ordinary skill in the art. The lamp itself is held in place by a pair of lampholders 33. The lampholders 33 provide both a mechanical means for securing the lamp 35 and electrical connectors for energizing the lamp arc 31, the means for energizing of means for killing the microorganisms.

As shown one side panel 34 holds each lampholder 33. The side panel 34 is preferably welded to the housing 60 the lampholders 33 preferably are of a snap in type for holding the lamp.

As shown, the reflector 40 is substantially parabolic over most of its surface. At the extremities the surface may be elliptical. As shown the reflector 40 is pressed against the walls 62 at the aperture 42 location and contacts the housing 60 internal surface creating a close fitting arrangement. The reflector is attached to the walls 62 by means of spot welds or threaded fasteners.

As shown, the emitted beams 30 will be arranged into an array of substantially parallel beams 32 which will be directed along a straight path portion of the straight prime conduit 22 of the ducting 80. As previously discussed, all of the features of this fourth embodiment apparatus are employed in the same way as the first, second and third embodiment to achieve this most effective and efficient method to kill microorganisms.

Although illustrative embodiments of the present invention have been described herein with reference to the accompanying drawings, it is to be understood that the invention is not limited to those precise embodiments, and that various other changes and modifications may be effected therein by one skilled in the art without departing from the scope or spirit of the invention. Although not shown on fist through third preferred embodiment it is understood that similar ballast 66 and other associated wiring and electrical connections are used as is conventional and understood by those of ordinary skill in the art.
This invention is not limited to the sterilization of fluid medium, but can be applied also for other purposes, for example, for the destruction or removal of hazardous chemicals from air or water by ultraviolet radiation.

## Claims

1. A method for killing microorganisms in a flowing fluid medium using germicidal beams as a means for killing the microorganisms in a straight portion of the flow path comprising the steps of
providing a primary flow of a fluid medium containing particles and microorganisms;
providing germicidal beams, said beams being immersed in said fluid medium containing particles and microorganisms along a straight portion of the flow path;
characterized by the steps of
providing at least one means for transferring said germicidal beams, and at least one means for orienting said germicidal beams into an array of substantially parallel germicidal beams aligned along said straight portion of the flow path;
at least one of said means for transferring being immersed in said fluid medium;
providing a secondary flow of substantially particle free fluid, said secondary flow running along or flowing across the surface of the at least one of said means for transferring which is immersed in said fluid medium and establishing a substantially particulate free barrier environment maintaining the immersed means for transferring clean.

2. The method according to claim 1 characterized in that said fluid medium is air.

3. The method according to claim 1 or 2 characterized in that one of said means for transferring is a fluid impenetrable transmissible wall which imperviously separates said primary flow from said means for orienting.

4. The method according to claim 1 or 2 characterized by the step of filtering said secondary flow.

5. The method according to claim 4 characterized by the step of providing a secondary flow includes the step of supplying said secondary flow of substantially particulate free fluid from said primary flow of the fluid medium, the secondary fluid being the same fluid as the primary fluid.

6. The method according to claim 1 or 2 characterized in that said germicidal beams are emitted by one or more arcs of ultraviolet lamps.

7. The method according to claim 1 or 2 further characterized by the step of passing said primary flow along a path aligned with said array of substantially parallel germicidal beams, said path being of sufficient length to allow said array of parallel germicidal beams to kill microorganisms.

8. The method according to claim 6 further characterized by the step of providing a substantially parabolic reflector around each said ultraviolet lamp, as the means for orienting the germicidal beams.

9. The method according to claim 8 characterized by the step of providing each said reflector with an aperture for accepting at least a portion of said secondary flow of substantially particle free fluid medium and passing said portion of said secondary flow of substantially particle free fluid medium through each said reflector.

10. Apparatus for killing microorganisms (10, 100) in a primary flow of a fluid medium (11) using germicidal beams (30) as a means for killing microorganisms in a straight portion (22) of the flow of a fluid medium (11) containing particles and microorganisms, the apparatus comprising
a conduit means (20) for passing a primary flow of a fluid medium (11), the means for passing having a straight prime conduit portion (22);
at least one means for orienting (40) the means for killing microorganisms
at least one means for transferring (43, 50) said means for killing microorganisms in said fluid medium (11),
a means for energizing (31) said germicidal beams (30);
characterized by
the means for killing microorganisms, in use, being oriented in an array of substantially parallel germicidal beams (32) aligned along the flow path of the fluid medium (11) in a portion of the straight prime conduit (22); at least one of said means for transferring (43, 50) being immersed in said fluid medium (11); a conduit means (23, 72) for passing a secondary flow (12) of a substantially particle free portion of said fluid medium (11), wherein said means for passing (23, 72) causes said secondary flow (12) to run along or flow across the surface of said means for transferring (43, 50) to establish a substantially particulate free barrier environment maintaining clean said means for transferring (43, 50).

11. Apparatus according to claim 10 characterized in that said conduit means (23, 72) for passing said secondary flow (12) of said fluid medium (11) has a filter (25), said filter being sufficient to remove particles from said secondary flow (12).

12. Apparatus according to claim 10 characterized in that said means for energizing are one or more arcs (31) of ultraviolet lamps which emit ultraviolet beams (30).

13. Apparatus according to claim 12 characterized in that said means for orienting (40) orients the emission of the ultraviolet beams (30) into a substantially parallel array of beams (32), said means for orienting said ultraviolet beams (30) being a substantially parabolic reflector (40).

14. Apparatus according to claim 13 characterized in that said straight prime conduit portion (22) is of sufficient length to allow said array of beams of ultraviolet radiation (32) to kill microorganisms.

15. Apparatus according to claim 14 characterized in that said means for orienting (40) the emission of germicidal beams (30) is situated at the end of said straight prime conduit portion (22) and is faced towards the straight prime conduit portion (22) so that the array of germicidal beams (32) are directed down the length of the straight prime conduit portion (22).

16. Apparatus according to claim 15 characterized in that said conduit means (23, 72) for passing said secondary flow (12) is a secondary conduit, the inlet of said secondary conduit being connected to said conduit means (20) for passing the primary flow of fluid medium (11) and being open for entrance of said fluid medium (11).

17. Apparatus according to claim 14, characterized in that said means for orienting (40) is open to the straight prime conduit portion (22) at a first end and in that at a second end, said means for orienting (40) is open for passing said secondary flow (12) of fluid medium (11).

18. Apparatus according to claim 13, characterized in that said substantially parabolic reflector (40) has an aperture (42) open to said secondary flow (12).

19. Apparatus according to claim 15 or 16, characterized in that the means for transferring (43, 50) is a fluid impenetrable transmissible wall (43) which imperviously separates said means for orienting (40) from the conduit means (22).

## Patentansprüche

1. Verfahren zum Abtöten von Mikroorganismen in einem strömenden, fluiden Medium unter Verwendung von keimtötenden Strahlen zum Abtöten der Mikroorganismen in einem geraden Bereich des Strömungspfades, aufweisend die Schritte, bei denen
eine primäre Strömung eines fluiden Mediums, das Partikel und Mikroorganismen enthält, vorgesehen wird;
keimtötende Strahlen vorgesehen werden, wobei die Strahlen längs eines geraden Bereichs des Strömungspfades in das fluide Medium, das Partikel und Mikroorganismen enthält, eingetaucht sind;
gekennzeichnet durch die Schritte, bei denen
mindestens ein Mittel vorgesehen wird, um die keimtötenden Strahlen zu übertragen, und mindestens ein Mittel vorgesehen wird, um die keimtötenden Strahlen zu einer Anordnung von im wesentlichen parallelen, keimtötenden Strahlen zu orientieren, die längs des geraden Bereichs des Strömungspfades ausgerichtet sind;
mindestens eines der Übertragungsmittel in das fluide Medium eingetaucht wird;
eine sekundäre Strömung aus einem im wesentlichen partikelfreien Fluid vorgesehen wird, wobei die sekundäre Strömung längs der Oberfläche oder über die Oberfläche des mindestens einen Übertragungsmittels, das in das fluide Medium eingetaucht ist, strömt, und eine im wesentlichen partikelfreie Barrierenumgebung errichtet, die das eingetauchte Übertragungsmittel sauber hält.

2. Verfahren gemäß Anspruch 1, dadurch gekennzeichnet, daß das fluide Medium Luft ist.

3. Verfahren gemäß Anspruch 1 oder 2, dadurch gekennzeichnet, daß eines der Übertragungsmittel eine für das Fluid undurchdringliche, durchlässige Wand ist, welche die primäre Strömung auf undurchdringliche Weise von dem Orientierungsmittel trennt.

4. Verfahren gemäß Anspruch 1 oder 2, gekennzeichnet durch den Schritt, bei dem die sekundäre Strömung gefiltert wird.

5. Verfahren gemäß Anspruch 4, dadurch gekennzeichnet, daß der Schritt, bei dem eine sekundäre Strömung vorgesehen wird, den Schritt umfaßt, bei dem die sekundäre Strömung aus einem im wesentlichen partikelfreien Fluid von der primären Strömung aus dem fluiden Medium abgeleitet wird, wobei das sekundäre Fluid das gleiche Fluid wie das primäre Fluid ist.

6. Verfahren gemäß Anspruch 1 oder 2, dadurch gekennzeichnet, daß die keimtötenden Strahlen von einem oder mehr Lichtbögen ultravioletter Lampen ausgesandt werden.

7. Verfahren gemäß Anspruch 1 oder 2, weiterhin gekennzeichnet durch den Schritt, bei dem die primäre Strömung längs eines Pfades strömt, der entsprechend der Anordnung von im wesentlichen parallelen, keimtötenden Strahlen ausgerichtet ist, wobei der Pfad eine genügende Länge hat, um der Anordnung von parallelen, keimtötenden Strahlen zu ermöglichen, Mikroorganismen abzutöten.

8. Verfahren gemäß Anspruch 6, weiterhin gekennzeichnet durch den Schritt, bei dem ein im wesentlichen parabolischer Reflektor um jede ultraviolette Lampe herum als das Mittel zum Orientieren der keimtötenden Strahlen vorgesehen wird.

9. Verfahren gemäß Anspruch 8, gekennzeichnet durch den Schritt, bei dem jeder Reflektor mit einer Öffnung versehen wird, um mindestens einen Bereich der sekundären Strömung des im wesentlichen partikelfreien, fluiden Mediums aufzunehmen, und diesen Bereich der sekundären Strömung aus einem im wesentlichen partikelfreien, fluiden Medium durch jeden Reflektor strömen zu lassen.

10. Vorrichtung (10, 100) zum Abtöten von Mikroorganismen in einer primären Strömung aus einem fluiden Medium (11) unter Verwendung von keimtötenden Strahlen (30) als ein Mittel zum Abtöten von Mikroorganismen in einem geraden Bereich (22) der Strömung eines fluiden Mediums (11), das Partikel und Mikroorganismen enthält, aufweisend
ein Rohrmittel (20), um eine primäre Strömung aus einem fluiden Medium (11) weiterzuleiten, wobei das Weiterleitungsmittel einen geraden Hauptrohrbereich (22) hat;
mindestens ein Mittel (40) zum Orientieren des Mittels zum Abtöten von Mikroorganismen;
mindestens ein Mittel (43, 50) zum Übertragen des Mittels zum Abtöten von Mikroorganismen in dem fluiden Medium (11);
ein Mittel (31) zum Erregen der keimtötenden Strahlen (30);
dadurch gekennzeichnet, daß
das Mittel zum Abtöten von Mikroorganismen bei der Verwendung in einer Anordnung von im wesentlichen parallelen, keimtötenden Strahlen (32) orientiert ist, die längs des Strömungspfades des fluiden Mediums (11) in einem Bereich des geraden Hauptrohrs (22) ausgerichtet sind; mindestens eines der Übertragungsmittel (43, 50) in das fluide Medium (11) eingetaucht ist; ein Rohrmittel (23, 72) zum Weiterleiten einer sekundären Strömung (12) aus einem im wesentlichen partikelfreien Bereich des fluiden Mediums (11) vorgesehen ist, wobei das Weiterleitungsmittel (23, 72) bewirkt, daß die sekundäre Strömung (12) längs der Oberfläche oder über die Oberfläche des Übertragungsmittels (43, 50) strömt, um eine im wesentlichen partikelfreie Barrierenumgebung zu errichten, die das Übertragungsmittel (43, 50) sauber hält.

11. Vorrichtung gemäß Anspruch 10, dadurch gekennzeichnet, daß das Rohrmittel (23, 72) zum Weiterleiten der sekundären Strömung (12) des fluiden Mediums (11) ein Filter (25) hat, wobei das Filter ausreicht, um Partikel aus der sekundären Strömung (12) zu entfernen.

12. Vorrichtung gemäß Anspruch 10, dadurch gekennzeichnet, daß die Erregungsmittel ein oder mehr Lichtbögen (31) von ultravioletten Lampen sind, die ultraviolette Strahlen (30) aussenden.

13. Vorrichtung gemäß Anspruch 12, dadurch gekennzeichnet, daß die Orientierungsmittel (40) die Aussendung der ultravioletten. Strahlen (30) zu einer im wesentlichen parallelen Anordnung von Strahlen (32) orientieren, wobei die Mittel zum Orientieren der ultravioletten Strahlen (30) ein im wesentlichen parabolischer Reflektor (40) sind.

14. Vorrichtung gemäß Anspruch 13, dadurch gekennzeichnet, daß der gerade Hauptrohrbereich (22) eine genügende Länge hat, um zu ermöglichen, daß die Anordnung von Strahlen (32) aus ultravioletter Strahlung Mikroorganismen abtötet.

15. Vorrichtung gemäß Anspruch 14, dadurch gekennzeichnet, daß das Mittel (40) zum Orientieren der Aussendung von keimtötenden Strahlen (30) an dem Ende des geraden Hauptrohrbereichs (22) gelegen ist und zu dem geraden Hauptrohrbereich (22) hin gerichtet ist, so daß die Anordnung von keimtötenden Strahlen (32) in Richtung der Länge des geraden Hauptrohrbereichs (22) gerichtet ist.

16. Vorrichtung gemäß Anspruch 15, dadurch gekennzeichnet, daß das Rohrmittel (23, 72) zum Weiterleiten der sekundären Strömung (12) ein sekundäres Rohr ist, wobei der Einlaß des sekundären Rohrs mit dem Rohrmittel (20) zum Weiterleiten der primären Strömung des fluiden Mediums (11) verbunden ist, und für das Einströmen des fluiden Mediums (11) offen ist.

17. Vorrichtung gemäß Anspruch 14, dadurch gekennzeichnet, daß das Orientierungsmittel (40) an einem ersten Ende zu dem geraden Hauptrohrbereich (22) hin offen ist, und daß das Orientierungsmittel (40) an einem zweiten Ende offen ist, um die sekundäre Strömung (12) des fluiden Mediums (11) hindurchzulassen.

18. Vorrichtung gemäß Anspruch 13, dadurch gekennzeichnet, daß der im wesentlichen parabolische Reflektor (40) eine zu der sekundären Strömung (12) hin offene Öffnung (42) hat.

19. Vorrichtung gemäß Anspruch 15 oder 16, dadurch gekennzeichnet, daß das Übertragungsmittel (43, 50) eine für das Fluid undurchdringliche, durchlässige Wand (43) ist, die das Orientierungsmittel (40) auf undurchdringliche Weise von dem Rohrmittel (22) trennt.

## Revendications

1. Procédé de destruction des micro-organismes dans un milieu fluide en mouvement utilisant des faisceaux germicides comme moyen de destruction des micro-organismes situés dans une partie droite du chemin d'écoulement, comprenant les étapes consistant à:
prévoir un écoulement primaire d'un milieu fluide contenant des particules et des micro-organismes;
prévoir des faisceaux germicides, lesdits faisceaux étant immergés dans ledit milieu fluide contenant des particules et des micro-organismes le long d'une partie droite du chemin d'écoulement;
caractérisé par les étapes consistant à:
prévoir au moins un moyen de transfert desdits faisceaux germicides, et au moins un moyen pour orienter lesdits faisceaux germicides en une rangée de faisceaux germicides sensiblement parallèles alignés le long de ladite partie droite du chemin d'écoulement;
au moins un desdits moyens de transfert étant immergé dans ledit milieu fluide;
prévoir un écoulement secondaire d'un fluide pratiquement dépourvu de particules, ledit écoulement secondaire circulant le long de ou s'écoulant à travers la surface de l'au moins un desdits moyens de transfert immergé dans ledit milieu fluide, et établissant un environnement formant barrage pratiquement dépourvu de particules permettant ainsi au moyen de transfert immergé de rester propre.

2. Procédé selon la revendication 1, caractérisé en ce que ledit milieu fluide est l'air.

3. Procédé selon la revendication 1 ou 2, caractérisé en ce que l'un desdits moyens de transfert est une paroi transmissible impénétrable par le fluide, celle-ci séparant de manière imperméable ledit écoulement primaire desdits moyens d'orientation.

4. Procédé selon la revendication 1 ou 2, caractérisé par l'étape consistant à filtrer ledit écoulement secondaire.

5. Procédé selon la revendication 4, caractérisé en ce que l'étape consistant à prévoir un écoulement secondaire comprend l'étape consistant à produire ledit écoulement secondaire de fluide pratiquement dépourvu de particules à partir dudit écoulement primaire du milieu fluide, le fluide secondaire étant identique au fluide primaire.

6. Procédé selon la revendication 1 ou 2, caractérisé en ce que les faisceaux germicides sont émis par un ou plusieurs arcs de lampes ultraviolettes.

7. Procédé selon la revendication 1 ou 2, caractérisé en outre par l'étape consistant à faire passer ledit écoulement primaire le long d'un chemin aligné avec ladite rangée de faisceaux germicides sensiblement parallèles, ledit chemin ayant une longueur suffisante pour permettre à ladite rangée de faisceaux germicides parallèles de détruire les micro-organismes.

8. Procédé selon la revendication 6, caractérisé en outre par l'étape consistant à prévoir un réflecteur sensiblement parabolique autour de chacune desdites lampes ultraviolettes, comme moyen d'orientation des faisceaux germicides.

9. Procédé selon la revendication 8, caractérisé par l'étape consistant à doter chacun desdits réflecteurs d'une ouverture, de façon à accepter au moins une partie dudit écoulement secondaire de milieu fluide pratiquement dépourvu de particules, et à faire passer ladite partie dudit écoulement secondaire de milieu fluide pratiquement dépourvu de particules à travers chacun desdits réflecteurs.

10. Dispositif de destruction des micro-organismes (10, 100) situé dans un écoulement primaire d'un milieu fluide (11), et utilisant des faisceaux germicides (30) comme moyens de destruction des micro-organismes dans une partie droite (22) de l'écoulement d'un milieu fluide (11) contenant des particules et des micro-organismes, le dispositif comprenant:
un moyen formant conduit (20) permettant le passage d'un écoulement primaire d'un milieu fluide (11), le moyen de passage possédant une partie droite formant conduit principal (22);
au moins un moyen d'orientation (40) des moyens de destruction des micro-organismes,
au moins un moyen de transfert (43, 50) des moyens de destruction de micro-organismes dans ledit milieu fluide (11),
un moyen d'excitation (31) desdits faisceaux germicides (30);
caractérisé en ce que:
en fonctionnement, le moyen de destruction des micro-organismes est orienté sous la forme d'une rangée de faisceaux germicides sensiblement parallèles (32) alignés le long du chemin d'écoulement du milieu fluide (11) dans une partie du conduit principal droit (22); au moins un desdits moyens de transfert (43, 50) est immergé dans ledit milieu fluide (11); par un moyen formant conduit (23, 72) permettant le passage d'un écoulement secondaire (12) d'une partie pratiquement dépourvue de particules dudit milieu fluide (11), ledit moyen de passage (23, 72) amenant ledit écoulement secondaire (12) à circuler le long de ou à s'écouler à travers la surface dudit moyen de transfert (43, 50), afin d'établir un environnement formant barrage pratiquement dépourvu de particules permettant audit moyen de transfert (43, 50) de rester propre.

11. Dispositif selon la revendication 10, caractérisé en ce que ledit moyen formant conduit (23, 72) de passage dudit écoulement secondaire (12) dudit milieu fluide (11) possède un filtre (25), ledit filtre étant suffisant pour retirer les particules dudit écoulement secondaire (12).

12. Dispositif selon la revendication 10, caractérisé en ce que ledit moyen d'excitation est constitué d'un ou plusieurs arcs (31) de lampes ultraviolettes qui émettent des faisceaux ultraviolets (30).

13. Dispositif selon la revendication 12, caractérisé en ce que ledit moyen d'orientation (40) oriente l'émission des faisceaux ultraviolets (30) sous la forme d'une rangée de faisceaux sensiblement parallèles (32), ledit moyen d'orientation desdits faisceaux ultraviolets (30) étant un réflecteur sensiblement parabolique (40).

14. Dispositif selon la revendication 13, caractérisé en ce que ladite partie droite formant conduit principal (22) a une longueur suffisante pour permettre à ladite rangée de faisceaux de rayons ultraviolets (32) de détruire les micro-organismes.

15. Dispositif selon la revendication 14, caractérisé en ce que ledit moyen d'orientation (40) de l'émission des faisceaux germicides (30) est situé à l'extrémité de ladite partie droite formant conduit principal (22) et est tourné vers la partie droite formant conduit principal (22), de sorte que la rangée de faisceaux germicides (32) est dirigée longitudinalement par rapport à la partie droite formant conduit principal (22).

16. Dispositif selon la revendication 15, caractérisé en ce que ledit moyen formant conduit (23, 72) de passage dudit écoulement secondaire (12) est un conduit secondaire, l'entrée dudit conduit secondaire étant reliée audit moyen formant conduit (20) de passage dudit écoulement primaire du milieu fluide (11), et étant ouverte afin de permettre l'entrée dudit milieu fluide (11).

17. Dispositif selon la revendication 14, caractérisé en ce que ledit moyen d'orientation (40) est ouvert vers la partie droite formant conduit principal (22) au niveau de sa première extrémité, et en ce qu'à sa seconde extrémité, ledit moyen d'orientation (40) est ouvert pour permettre le passage dudit écoulement secondaire (12) du milieu fluide (11).

18. Dispositif selon la revendication 13, caractérisé en ce que ledit réflecteur sensiblement parabolique (40) possède une ouverture (42) ouverte sur ledit écoulement secondaire (12).

19. Dispositif selon la revendication 15 ou 16, caractérisé en ce que le moyen de transfert (43, 50) est une paroi transmissible impénétrable par le fluide (43), celle-ci séparant de façon imperméable ledit moyen d'orientation (40) du moyen formant conduit (22).
